# EUROPEAN PATENT SPECIFICATION

(11) **EP 1 552 814 B1**
(45) Date of publication and mention of the grant of the patent: **10.12.2008**
(21) Application number: 03784610.2
(22) Date of filing: 08.08.2003
(51) Int. Cl.: A61K 8/37, A61Q 15/00, A61Q 13/00

(54) **FRAGRANCE COMPOSITION**
DUFTZUSAMMENSETZUNG
COMPOSITION DE FRAGRANCE

(30) Priority: 09.08.2002 JP 2002234104
(43) Date of publication of application: 13.07.2005
(60) Divisional application: 08166557.2
(73) Proprietor: KAO CORPORATION, Tokyo 103-8210 (JP)
(72) Inventor: ISHIHARA, Hideki, KAO CORPORATION RESEARCH LAB, Tokyo 131-8501 (JP); FUJIHARA, Hirohisa, KAO CORPORATION RESEARCH LAB., Tokyo 131-8501 (JP); TAKADA, Yoshihiro, KAO CORPORATION RESEARCH LAB., Tokyo 131-8501 (JP); TERAZAKI, Hiroyuki, KAO CORPORATION RESEARCH LAB., Tokyo 131-8501 (JP)
(74) Representative: HOFFMANN EITLE
(86) International application number: PCT/JP2003/010136
(87) International publication number: WO 2004/014332

(56) References cited:
- EP-A- 1 213 276
- WO-A-03/088933
- WO-A1-98/50011
- WO-A2-01/43784
- GB-A- 730 095
- JP-A- 5 310 543
- JP-A- 11 139 923
- JP-A- 2000 191 448
- JP-A- 2001 064 120
- JP-A- 2002 053 435
- US-A- 3 637 533
- US-A- 4 318 831
- US-A- 4 331 569
- US-A- 5 089 162
- US-A- 5 135 747
- US-A- 5 540 853
- CRUPI ET AL: 'A comprehensive study on the chemical composition and aromatic characteristics of lemon liquor' FOOD CHEMISTRY vol. 105, no. 2, 19 July 2007, ELSEVIER SCIENCE PUBLISHERS LTD, GB, pages 771 - 783, XP022163364

## Description

### Field of the Invention

This invention relates to fragrance compositions which exhibit a high masking effect on acid smell, retain good long-term stability at high temperatures, give off a good scent from a bottle in which the hair cosmetic composition is filled as well as a good scent upon application of the hair cosmetic composition and are excellent in the persistence of such scent after the application, even when added to a hair cosmetic composition having a pH of 1 to 5.

### Background of the Invention

Conventional hair cosmetic compositions generally have a pH in the neutral range and contain little acid therein, so no problem has been manifested about an acid smell from a cosmetic base. Further, no attention has been paid to fragrances added to hair cosmetic preparations or their stability in the acidic range.

With the consumers' needs changing in recent years, developments have been made to formulate hair cosmetic compositions having a pH in the acidic range such that various functions can be imparted to them. Acidic hair cosmetic compositions, however, give off a peculiar acid smell. A need has, therefore, arisen to mask a smell of a cosmetic base contained in acidic hair cosmetic compositions.

Acidic hair cosmetic compositions are low in pH. When fragrances added to conventional hair cosmetic compositions are simply added to such acidic hair cosmetic compositions, they develop such a problem that an odor balance deteriorates and an unpleasant odor manifests (JP-A-2000-143453).

JP-A-2001-64120 and JP-A-2002-53435 describe hair cosmetic compositions containing fragrance compositions comprising a musk, an ester compound, and a hydrocarbon.

US-A-5,540,853 describes hair cosmetic compositions containing a fragrance composition comprising a musk and an ester compound.

The present invention provides a fragrance composition which can mask a smell peculiar to an acidic hair cosmetic composition and is also excellent in long-term stability. The present invention also provides a fragrance composition which gives off a good scent from a bottle in which a hair cosmetic composition is filled, as well as a good scent upon application of the hair cosmetic composition, and is excellent in the persistence of such scent after the application.

### Disclosure of the Invention

The present inventors, therefore, have proceeded with various investigations. As a result, it has been found that a combination of a musk with one or more ingredients of specific chemical structures in particular proportions makes it possible to obtain a fragrance composition which can mask a smell peculiar to an acidic hair cosmetic composition and is also excellent in long-term stability.

Described specifically, the present invention provides a hair cosmetic composition having a pH of 1 to 5, which comprises the following ingredients (I) to (IV):
(I) from 1 to 50 wt.% of an anionic surfactant;
(II) from 0.05 to 10 wt.% of oil;
(III) from 0.05 to 10 wt.% of an organic acid; and
(IV) a fragrance composition comprising the following ingredient (A), (B) and (C):
   (A) from 0.1 to 70 wt.%, with respect to the total weight of the fragrance composition, of a musk;
   (B) from 0.001 to 80 wt.%, with respect to the total weight of the fragrance composition, of a compound represented by the following formula(1): R¹-COOR² (1)
      wherein R¹ represents a linear, branched or cyclic hydrocarbon group having from 2 to 14 carbon atoms, which may contain an oxygen atom or nitrogen atom in at least one carbon-to-carbon bond thereof, and R² represents a linear, branched or cyclic hydrocarbon group having from 1 to 15 carbon atoms, which may contain an oxygen atom or nitrogen atom in at least one carbon-to-carbon bond thereof; and
   (C) from 0.01 to 90 wt.%, with respect to the total weight of the fragrance composition, of a hydrocarbon having a total carbon number of from 5 to 15.

For a fragrance composition to be added to an acidic hair cosmetic composition having a pH of 1 to 5, on the other hand, it is important as basic requirements to mask an acid smell derived from a cosmetic base and to have good long-term storage stability. Further, from the viewpoint of its comfortable use by consumers and the enhancement of its attraction as merchandise, it is also important that the hair cosmetic composition has a good scent at each of the following three stages:
1. The scent emitted from the bottle mouth is favorable since this serves as one of the important factors for consumers upon selecting a brand at the stores.
2. The scent upon its application is favorable.
3. A favorable scent remains at an adequate intensity on the hair after the application.

As the musk employed as ingredient (A) in the fragrance composition according to the present invention, a synthetic musk can be mentioned. Specific examples include muscone, civetone, cyclopentadecanone, 5-cyclohexadecen-1-one, cyclopentadecanolide, ambrettolide, 12-ketocyclopentadecanolide, cyclohexadecanolide, 7-cyclohexadecanolide, 12-oxa-16-hexadecanolide, 11-oxa-16-hexadecanolide, 10-oxa-16-hexadecanolide, ethylene brassylate, 3-methycyclopentadecenone (muscenone, NF), cyclopentadenolide (pentalide), ethylenedodecane dioate, musk ketone, 6-acetylhexamethylindan ("PHANTOLID", trade name of PFW Aroma Chemicals B.V.), 4-acetyldimethyl-t-butylindan ("CELESTOLIDE", trade name of International Flavors & Fragrances Inc.), 5-acetyltetramethylisopropylindan ("TRASEOLIDE", trade name of Quest International B.V.), 6-acetylhexatetralin ("TENTAROME", trade name of PFW Aroma Chemicals B.V.), tetramethyl-6-ethyl-7-acetyl-tetrahydronaphthalene ("VERSALIDE", trade name of Givaudan-Roure Corporation), formylethyltetramethyltetralin, acetyldimethyltetrahydrobenzindanone ("VITALIDE", trade name of Takasago International Corporation), hexamethylhexahydrocyclopentabenzopyran ("GALAXOLIDE", trade name of International Flavors & Fragrances Inc.), and 3-methylcyclopentadeceone ("MUSCENONE DELTA", Firmenich, Inc.). Among these synthetic musks, preferred examples are muscone, ambrettolide, ethylene brassylate, musk ketone, 3-methylcyclopentadecenone (muscenone, NF), cyclopentadecenolide (pentalide), hexamethylhexahydrocyclopentabenzopyran ("GALAXOLIDE", trade name of International Flavors & Fragrances Inc.), and 3-methylcyclopentadeceone ("MUSCENONE DELTA", Firmenich, Inc.).

From the viewpoint of including an amount sufficient to mask an acid smell, ensuring a harmony in fragrance with other materials and imparting mildness to fragrance, the content of the musk is from 0.1 to 70 wt.%, preferably from 1 to 50 wt. %, more preferably from 2 to 40 wt. % of the fragrance composition.

Ingredient (B) employed in the present invention is a compound of formula (1).

In formula (1), the groups represented by R¹ and R² can each be a linear, branched or cyclic hydrocarbon group, or a group containing a linear, branched or cyclic hydrocarbon group with an oxygen atom or nitrogen atom inserted in at least one carbon-to-carbon bond thereof. It is to be noted that the term "hydrocarbon group" as used herein includes both saturated and unsaturated ones and the term "cyclic hydrocarbon group" as used herein includes saturated, unsaturated and aromatic, cyclic hydrocarbon groups. As the atom inserted in the at least one carbon-to-carbon bond, an oxygen atom or a nitrogen atom can bementioned, with an oxygen atom being preferred. A preferred form of linkage with an oxygen atom contained therein is an ether linkage in a linear ether or cyclic ether. R¹ has from 2 to 14 carbon atoms, while R² has from 1 to 15 carbon atoms.

Preferred examples of R¹ and R² include alkyl groups, alkenyl groups, cyclic hydrocarbon groups, cyclic hydrocarbyl-alkyl groups, cyclic hydrocarbyl-alkenyl groups, aromatic hydrocarbon groups, aromatic hydrocarbyl-alkyl groups, aromatic hydrocarbyl-alkenyl groups, and monoterpene and other terpene groups.

Examples of the compound of formula (1) include terpenyl esters, aliphatic esters, and aromatic esters. Illustrative of the terpenyl esters of formula (1) are citronellyl propionate, geranylpropionate, linalylpropionate, terpinyl propionate, rhodinyl propionate, neryl propionate, carbinyl propionate, menthyl propionate, bornyl propionate, isobornyl propionate, linalyl butyrate, geranyl butyrate, citronellyl butyrate, rhodinyl butyrate, neryl butyrate, terpenyl butyrate, santalyl butyrate, citronellyl isobutyrate, geranyl isobutyrate, linalyl isobutyrate, rhodinyl isobutyrate, neryl isobutyrate, terpinyl isobutyrate, linalyl isovalerate, citronellyl isovalerate, geranyl isovalerate, menthyl isovalerate, terpinyl isovalerate, linalyl hexanoate, citronellylhexanoate, geranyl hexanoate, linalyl octanoate, citronellyl tiglate, geranyl benzoate, linalyl benzoate, geranyl phenylacetate, citronellyl phenylacetate, rhodinylphenylacetate,menthylphenylacetate, linalyl cinnamate, citronellyl tiglate, geranyl tiglate, methyl geranate, ethyl geranate, methyl cyclogeranate, ethyl cyclogeranate, and ethylcitronellyl oxalate.

Examples of the aliphatic esters of formula (1) include ethyl propionate, propyl propionate, allyl propionate, butyl propionate, isobutyl propionate, isoamyl propionate, hexyl propionate, cis-3-hexenyl propionate, trans-2-hexenyl propionate, decenyl propionate, tricyclodecenyl propionate, methyl butyrate, ethyl butyrate, propyl butyrate, isopropyl butyrate, allyl butyrate, butyl butyrate, isobutyl butyrate, amyl butyrate, isoamyl butyrate, hexyl butyrate, heptyl butyrate, cis-3-hexenyl butyrate, trans-2-hexenyl butyrate, octyl butyrate, propylene glycol dibutyrate, cyclohexyl butyrate, tetrahydrofurfuryl butyrate, methyl isobutyrate, ethyl isobutyrate, propyl isobutyrate, isopropyl isobutyrate, butyl isobutyrate, isobutyl isobutyrate, isoamyl isobutyrate, hexyl isobutyrate, cis-3-hexenyl isobutyrate, 2,4-hexadienyl isobutyrate, 1,3-dimethyl-3-butenyl isobutyrate, octyl isobutyrate, tricyclodecenyl isobutyrate, methyl 2-methylbutyrate, ethyl 2-methylbutyrate, 2-methylbutyl 2-methylbutyrate, hexyl 2-metylbutyrate, cis-3-hexenyl 2-methylbutyrate, allyl 2-ethylbutyrate, ethyl 3-hydroxybutyrate, methyl valerate, ethyl valerate, propyl valerate, butyl valerate, isobutyl valerate, amyl valerate, cis-3-hexenyl valerate, methyl isovalerate, ethyl isovalerate, propyl isovalerate, isopropyl isovalerate, allyl isovalerate, butyl isovalerate, isobutyl isovalerate, isoamyl isovalerate, 2-methylbutyl isovalerate, hexyl isovalerate, heptyl isovalerate, ethyl tricyclo [5.2.1.0^{2,6}]decan-2-yl carboxylate ("FRUITATE", Kao Corporation), and ethyl 2-cyclohexylpropionate ("POIRENATE", Kao Corporation). Preferred are butyl propionate, ethyl butyrate, propyl butyrate, isopropyl butyrate, butyl butyrate, isobutyl butyrate, amyl butyrate, isoamyl butyrate, cis-3-hexenyl butyrate, ethyl isobutyrate, butyl isobutyrate, isobutyl isobutyrate, ethyl 2-methylbutyrate, 2-methylbutyl 2-methylbutyrate, ethyl valerate, butyl valerate, isobutyl valerate, amyl valerate, ethyl isovalerate, butyl isovalerate, isobutyl isovalerate, isoamyl isovalerate, 2-methylbutyl isovalerate, ethyl tricyclo[5.2.1.0^{2,6}]decan-2-yl carboxylate ("FRUITATE", Kao Corporation), and ethyl 2-cyclohexylpropionate ("POIRENATE", Kao Corporation).

Examples of the aromatic esters of formula (1) include ethyl benzylacetoacetate, benzyl propionate, styralyl propionate, anisyl propionate, phenylethyl propionate, cinnamyl propionate, phenylpropyl propionate, dimethylbenzylcarvinyl propionate, phenoxyethyl propionate, propylene glycol dipropionate, ethyl 3-hydroxy-3-phenylpropionate, isobutyl furanpropionate, benzyl butyrate, cinnamyl butyrate, phenylethyl butyrate, dimethylbenzylcarvinyl butyrate, benzyl isobutyrate, p-cresyl isobutyrate, cinnamyl isobutyrate, phenylethyl isobutyrate, phenoxyethyl isobutyrate, phenylpropyl isobutyrate, styrallyl isobutyrate, dimethylbenzylcarvinyl isobutyrate, dimethylpheylethylcarvinyl isobutyrate, decahydro-β-naphthyl isobutyrate, benzyl 2-methylbutyrate, phenylethyl 2-methylbutyrate, benzyl valerate, phenylethyl valerate, furfuryl valerate, benzyl isobutyrate, cinnamyl isovalerate, phenylethyl isovalerate, phenylpropyl isovalerate, benzyl hexanoate, benzyl octanoate, phenylethyl octanoate, p-cresyl octanoate, phenylethyl nonaoate, benzyl dodecanoate (benzyl laurate), methyl benzoate, ethyl benzoate, propyl benzoate, isopropyl benzoate, allyl benzoate, isobutyl benzoate, isoamyl benzoate, prenyl benzoate, hexyl benzoate, cis-3-hexenyl benzoate, benzyl benzoate, phenylethyl benzoate, ethyl o-methoxybenzoate, methyl dihydroxydimethylbenzoate, methyl phenylacetate, ethyl phenylacetate, propylphenylacetate, isopropylphenylacetate, butyl phenylacetate, isobutyl phenylacetate, isoamyl phenylacetate, hexyl phenylacetate, cis-3-hexenyl phenylacetate, benzyl phenylacetate, phenylethyl phenylacetate, p-cresylphenylacetate, eugenylphenylacetate, isoeugenyl phenylacetate, methyl cinnamate, ethyl cinnamate, propylcinnamate, isopropyl cinnamate, allyl cinnamate, isobutyl cinnamate, isoamyl cinnamate, benzyl cinnamate, cinnamyl cinnamate, phenylethyl cinnamate, dimethyl phthalate, diethyl phthalate, methyl salicylate, ethyl salicylate, butyl salicylate, isobutyl salicylate, amyl salicylate, isoamyl salicylate, allyl salicylate, hexyl salicylate,cis-3-hexenyl salicylate, cyclohexyl salicylate, phenylsalicylate,benzylsalicylate,phenylethylsalicylate, p-cresyl salicylate, allyl phenoxyacetate, ethyl phenylpropionate, benzyl tiglate, phenylethyl tiglate, cinnamyl tiglate, benzyl angelate, phenylethyl angelate, cinnamyl angelate, and phenyl angelate.

Preferred are benzyl isovalerate, cinnamyl isovalerate, phenylethyl isovalerate, ethyl benzoate, propyl benzoate, isopropyl benzoate, allyl benzoate, isobutyl benzoate, isoamyl benzoate, prenyl benzoate, hexyl benzoate, cis-3-hexenyl benzoate, benzyl benzoate, phenylethyl benzoate, methyl cinnamate, ethyl cinnamate, methyl salicylate, ethyl salicylate, amyl salicylate, isoamyl salicylate, hexyl salicylate, and cis-3-hexenyl salicylate.

The content of ingredient (B)(i) is from 0.001 to 80 wt.%, preferably from 1 to 80 wt.%, more preferably from 1.5 to 60 wt.% of the fragrance composition.

These ingredients (B) can be used either singly or in combination. Further, the content of ingredient (B) is from 0.001 to 80 wt. % of the fragrance composition from the viewpoint of improvements in scent and tastefulness.

Ingredient (C), i.e., the hydrocarbon having a total carbon number of from 5 to 15 can be a terpenyl hydrocarbon. Illustrative are α-pinene, β-pinene, camphene, myrcene, dihydromyrcene, limonene, dipentene, terpinene,terpinolene, carene, allo-ocimene, ocimene, α-phellandrene, p-cymene, β-caryophyllene, β-farnesene, bisabolene, cedrene, cadinene, valencene, tsujopsene, da-i-ene, andlongifolene. Preferred are α-pinene, β-pinene, and limonene.

These ingredients (C) can be used either singly or in combination, although it is preferred to use two or more of them. The content of ingredient (C) in the fragrance composition differs depending upon the kinds and combination of ingredients used, but can be preferably from 0.001 to 90 wit. %, more preferably from 0.01 to 70 wit. %, even more preferably from 0.1 to 40 wt.% from the standpoint of amount sufficient to mask an acid smell, ensuring a balance with other materials, and achieving improvements in scent and tastefulness.

From the standpoint of enhancing the scent emmited, making improvements in the refreshing sensation and to give a more defined body for the fragrance, it is preferred to incorporate, in addition to the above-described ingredients, a sulfur-containing compound as an ingredient (D) in the fragrance composition according to the present invention. As the sulfur compounds, an organosulfur compound such as a thiol compound, sulfide compound, disulfide compound, thioaldehyde compound or cyclic thioether compound can be mentioned. Specific examples include propyl mercaptan, isopropyl mercaptan,2-methyl-3-buthanethiol,allylmercaptan, isoamyl mercaptan, thiogeraniol, limonenethiol, 8-mercaptomenthone (sulfox),phenylmercaptan,o-thiocresol, 2-ethylthiophenol, 2-naphthylmercaptan, furfuryl mercaptan, 2-methyl-3-franthiol, dimethyl sulfide, dimethyl disulfide, dimethyl trisulfide, methylpropyl disulfide, methylpropyl trisulfide, propyl disulfide, dipropyl trisulfide, diallyl trisulfide, diallyl disulfide, dibutyl sulfide, methionol, 3-methylthio-1-hexanol, methional, mentho sulfide, dithiospirolactone, furfurylmethyl sulfide, 2-methyl-5-methylthiofuran, methylfuryl disulfide, furfuryl disulfide, thiophene, tetrahydrothiophene, 3-thiophenecarboxaladehyde, 5-methyl-2-thiophenecarboxaldehyde, tetrahydrothiophen-3-one, trithioacetone, 2-methyl-4-propyl-1,3-oxathiane, thioglycolic acid, methyl ethylthioacetate, ethyl methylthioacetate, 2-methylmercaptopropionic acid, pineapple mercaptan, ethyl 3-methylthiopropionate, ethyl thioacetate, furfuryl thioacetate, furfuryl thiopropionate, methyl thiobutyrate, methylmethane thiosulfonate, allyl isothiocyanate, benzyl isothianate, thialdine, 2-methyl-4-propyl-1,3-oxathiane, p-menthane-8-thiol-3-one, p-mentene-8-thiol, and methyl β-methylthiopropionate.

From the standpoint of the amount sufficient to mask an acid smell and ensuring a balance with other materials, the content of the sulfur compound in the fragrance composition can be preferably from 0.00001 to 1 wt. %, more preferably from 0.0001 to 0.5 wt.%, even more preferably from 0.0002 to 0.4 wt.%.

The fragrance composition according to the present invention can also contain 1-(2-t-butylcyclohexyloxy)-2-butanol, dodecahydro-3a,6,6,9a-tetramethyl-naphtho[2.1-b]furan, 2-ethoxynaphthalene, 2-methoxynaphthalene, 1H-3a,7-methanoazulene, octahydro-6-methoxy-3,6,8,8-tetramethyl-, [3R-(3α,3aβ,6β,7β,8aα)], 2-oxybicyclo[2.2.2]octane, 1,3,3-trimethyl, 3,7,-dimethyl-2,6-octadienenitrile, 4,4a,5,9b-tetrahydroindeno[1,2d] -1,3-dioxine, tetrahydro-4-methyl-2-(2-methyl-1-propenyl)-2H-pyran, cyclohexanol, 3-(5,5,6-trimethylbicyclo[2.2.1]hept-2-yl)-cyclohexanol, 2-tridecenenitrile, 2-methoxy-4-allylphenol, 3-methyl-5-phenyl-1-pentanol, 1-(2-t-butylcyclohexyloxy)-2-butanol, and/or 2-methyl-4-(2,2,3-trimethyl-3-cyclopentene-1-yl)-2-buten-1-ol. From the viewpoint of imparting distinctiveness to the fragrance, the content of such an additional fragrant material may preferably be from 0.001 to 50 wt.% of the fragrance composition.

In addition, the fragrance composition according to the present invention can also contain one or more of alcohols, polyhydric alcohols and ethers.

According to the present invention, a hair cosmetic composition is formulated with the above-described fragrance composition contained therein. As the fragrance composition to be used according to the present invention is excellent in long-term stability under high-temperature conditions and can mask a smell peculiar to an acidic hair cosmetic composition, it is useful as a fragrance composition for hair cosmetic compositions whose pHs are from 1 to 5, preferably from 2 to 4 (even more preferably, from 3 to 4). The hair cosmetic compositions of pH 1 to 5 according to the present invention can include hair cleansing compositions, rinses, treatments, conditioning agents, hair packs, hair creams, styling hair care products, hair tonics, hair restorers, hair colognes and the like, each of which has a pH of from 1 to 5, but excludes hair manicures, hair dyes, and permanent wave solutions. Among these, those used by washing them off, such as hair cleansing compositions, e.g., shampoos and conditioning shampoos, and hair rinses are preferred. It is to be noted that the term "hair cosmetic composition having a pH of 1 to 5" (25°C) as used herein means a hair cosmetic composition whose pH is from 1 to 5 when the undiluted hair cosmetic composition is diluted 20-fold with water.

A hair cosmetic composition having a pH of 1 to 5 can be prepared with a similar formula as an ordinary hair cosmetic composition except that its pH is controlled from 1 to 5. Accordingly, an oil ingredient, a conditioning agent, a humectant, a viscosity increasing agent, a viscosity controlling agent, an emulsifier, a colorant, a stabilizer, an ultraviolet absorber, a preservative, a pH adjuster and the like are added as needed in addition to one or more surfactants as a cleansing ingredient. As the surfactants, anionic surfactants are mentioned. Examples of the anionic surfactants include polyoxyethylene alkyl ether sulfates, polyoxyethylene alkenyl ether sulfates, alkyl sulfates, and polyoxyalkylene alkyl phenyl ether sulfates, especially sulfate-type anionic surfactants such as polyoxyethylene alkyl ether sulfates and alkyl sulfates; and sulfonates and carboxylates such as alkyl sulfosuccinate salts, polyoxyalkylene alkyl sulfosuccinate salts, higher fatty acid salts, and alkane sulfonate salts.

As the oil ingredients, higher alcohols, lanolins, liquid paraffin, higher fatty acids, ester oils, and silicones can be mentioned. Examples of the silicones can include the following silicones:

### (1) Dimethylpolysiloxanes

Those represented by the following formula can be mentioned as examples.

(Me)₃Si-[(Me)₂SiO]_{d}-Si(Me)₃

wherein Me represents a methyl group, and d stands for a value of from 3 to 20,000.

### (2) Amino-modified silicones

Preferred examples are those having an average molecular weight of from about 3, 000 to 100, 000 and described under the name of amodimethicones in the third edition of the CTFA Cosmetic Ingredient Dictionary, U.S.A., although various amino-modified silicones are usable. Preferably, these amino-modified silicones are used as aqueous emulsions. As commercial products, "SM8704C" (product of Dow Corning Toray Silicone Co., Ltd.) and "DC929" (product of Dow Corning Corporation) can be mentioned.

### (3) Other silicones

In addition to the above-described silicones, polyether-modified silicones, methylphenylpolysiloxane, fatty-acid-modified silicones, alcohol-modified silicones, alkoxy-modified silicones, epoxy-modified silicones, fluorine-modified silicones, cyclic silicones, and alkyl-modified silicones can also be mentioned.

The oil ingredient is contained at from 0.05 to 10 wt.%, preferably at from 0.1 to 5 wt.%, more preferably at from 0.3 to 2 wt.% in the hair cosmetic composition.

As the conditioning agent, a cationic polymer is preferred. Examples of the cationic polymer include cationized cellulose derivatives, cationic starch, cationized guar gum derivatives, homopolymers of diallyl(quaternary ammonium) salts, diallyl(quaternary ammonium) salt/acrylamide copolymer, quaternized polyvinylpyrrolidone derivatives, polyglycol-polyamine condensation products, vinylimidazolium trichloride/vinylpyrrolidone copolymer, hydroxyethylcellulose/dimethyldiallylammonium chloride copolymer, vinylpyrrolidone/quaternized dimethylaminoethyl methacrylate copolymer, polyvinylpyrrolidone/alkyl aminoacrylate copolymers, polyvinylpyrrolidone/alkyl aminoacrylate/vinylcaprolactam copolymer, vinylpyrrolidone/methacrylamidopropyl-trimethylammonium chloride copolymer, alkylacrylamide/acrylate/alkylaminoalkyl acrylamide/polyethylene glycol methacrylate copolymers, adipic acid/dimethylaminohydroxypropyl ethylenetriamine copolymer ("CARTARETIN", product of Sandoz, Inc., U.S.A.), and cationic polymers disclosed in JP-A-53-139734 or JP-A-60-36407. Preferred example are cationized cellulose derivatives and cationized guar gum derivatives. The conditioning agent can be contained preferably at from 0.05 to 5 wt.%, more preferably at from 0.1 to 3 wt.%, even more preferably at form 0.3 to 1 wt.% in the hair cosmetic composition.

In the hair cosmetic composition according to the present invention, an organic acid is additionally incorporated to make improvements in the finish of hair such as sleekness and style. Examples of the organic acid include carboxylic acids such as monocarboxylic acids, dicarboxylic acids, hydroxycarboxylic acids and polycarboxylic acids, and alkylphosphoric acids. Among these, carboxylic acids, especially dicarboxylic acids and hydroxycarboxylic acids are preferred. Examples of the dicarboxylic acids include malonic acid, succinic acid, glutaric acid, adipic acid, maleic acid, fumaric acid, and phthalic acid, and illustrative of the hydroxycarboxylic acids are glycolic acid, lactic acid, hydroxyacrylic acid, oxybutyric acid, glyceric acid, malic acid, tartaric acid, and citric acid. Among these, α-hydroxycarboxylic acids, especially lactic acid and malic acid are preferred.

Two or more of these organic acids can be used in combination. Further, the content of the organic acid is from 0.05 to 10 wt.%, preferably from 0.1 to 5 wt. %, more preferably from 0.5 to 1 wt. % of the hair cosmetic composition according to the present invention.

In the hair cosmetic composition according to the present invention, an aromatic alcohol may be additionally incorporated to make improvements in touch feel and post-shampoo sleekness. Examples of the aromatic alcohol include benzyl alcohol, benzyloxyethanol and phenoxyethanol, with benzyl alcohol and benzyloxyethanol being preferred.

Two or more of these aromatic alcohols can be used in combination. Further, the content of the aromatic alcohol can be preferably from 0.01 to 20 wt.%, more preferably from 0.1 to 10 wt.%, even more preferably from 0.5 to 5 wt.% of the hair cosmetic composition according to the present invention.

The hair cosmetic composition having a pH of 1 to 5 according to the present invention can be produced in a similar manner as conventional hair cosmetic compositions except that its pH is controlled from 1 to 5.

### Examples

In the Examples, "%" means "wt.%", and "part" or "parts" means "part by weight" or "parts by weight".

### Example A Conditioning Shampoos

An unfragranced conditioning shampoo (pH 3.7) of the formula shown in Table 1 was prepared. Aliquots of the unfragranced conditioning shampoo were fragranced with 0.5 wt.% of the fragrance compositions 1 to 12 shown in Table 2 andTable3, respectively, to formulate conditioning shampoos. Samples of the conditioning shampoos were weighed as much as 20 g each in standard 50-mL wide-mouth bottles "PS-06" (made of clear glass), and were placed in a storage cabinet controlled at 50°C. One month later, a scent given off from the surface of each shampoo was ranked by two expert panelists in accordance with the below-described ranking standard, and the average of their ranking scores was recorded. The ranking was an overall ranking on a masking effect for an acid smell, stability and the like while taking into consideration the spreading characteristics, the floating pattern and the like of the fragrance.

### Ranking standard:

5: Excellent
4: Good
3: Satisfactory as a commercial product
2: Slightly poor
1: Poor

**Table 1**

| Conditioning shampoo composition (pH 3.7) | |
|---|---|
| | (wt.%) |
| Sodium POE(2) lauryl ether sulfate | 11.0 |
| Sodium lauryl sulfate | 5.0 |
| Cationized guar gum | 0.3 |
| Malic acid | 0.75 |
| Lactic acid | 0.1 |
| Sodium chloride | 0.2 |
| Benzyl alcohol | 0.5 |
| Cocoyl monoethanolamide | 1.0 |
| Dimethicone (viscosity: 100,000 cps) | 0.5 |
| Amodimethicone | 0.1 |
| Myristyl alcohol | 1.0 |
| Cetanol | 0.5 |
| Ethylene glycol distearate | 3.0 |
| Cationized hydroxyethylcellulose | 0.3 |
| Glycerol | 1.0 |
| Sodium hydroxide | q.s. to pH 3.7. |
| Deionized water | Balance |

**Table 2**

| Types | Ingredient names | Fragrance compositions | | | | | | |
|---|---|---|---|---|---|---|---|---|
| | | 1 | 2 | 3 | 4 | 5 | 6 | 7 |
| A:MUSK | Musk ketone | 0 | 3 | 3 | 3 | 3 | 3 | 3 |
| A:MUSK | "GALAXOLIDE" | 0 | 93 | 93 | 93 | 93 | 93 | 93 |
| B-i:ESTER | Cis-3-hexenyl salicylate | 0 | 0 | 15 | 0 | 0 | 15 | 15 |
| B-i:ESTER | Ethyl 2-methylbutyrate (DPG 10%) | 0 | 0 | 5 | 0 | 0 | 5 | 5 |
| B-i:ESTER | "FRUITATE" (ethyl tricyclodecanylcarboxylate) | 0 | 0 | 5 | 0 | 0 | 5 | 5 |
| B-i:ESTER | Tricyclodecenyl propionate | 0 | 0 | 20 | 0 | 0 | 20 | 20 |
| B-i:ESTER | Methyl salicylate (DPG 10%) | 0 | 0 | 5 | 0 | 0 | 5 | 5 |
| B-ii:ESTER | o-t-Butyl cyclohexylacetate | 0 | 0 | 15 | 0 | 0 | 15 | 15 |
| B-iii:KETONE | "ADMASCENONE" | 0 | 0 | 0 | 0.5 | 0 | 0 | 0.5 |
| B-iii:KETONE | "α-DAMASCONE" | 0 | 0 | 0 | 1.5 | 0 | 0 | 1.5 |
| B-iii:KETONE | β-Ionone | 0 | 0 | 0 | 20 | 0 | 0 | 20 |
| B-iii:KETONE | Methylionone-G | 0 | 0 | 0 | 25 | 0 | 0 | 25 |
| C:TERPENE | Limonene | 0 | 0 | 0 | 0 | 25 | 25 | 25 |
| SOLVENT | Dipropylene glycol | 350 | 244 | 191 | 207 | 229 | 164 | 117 |
| | FORMULA*^{note 1} | 650 | 650 | 650 | 650 | 650 | 650 | 650 |
| | Total (parts by weight) | 1000 | 1000 | 1000 | 1000 | 1000 | 1000 | 1000 |

| | | | | | | | | |
|---|---|---|---|---|---|---|---|---|
| FORMULA*^{note 1} Fragrance formula formed primarily of alcohols and containing aldehydes, dipropylene glycol, etc. | | | | | | | | |

**Table 3**

| Types | Ingredient names | Fragrance compositions | | | | |
|---|---|---|---|---|---|---|
| | | 8 | 9 | 10 | 11 | 12 |
| - | Hexyl acetate | 5 | 0 | 0 | 0 | 0 |
| - | Iso-amyl acetate | 2 | 0 | 0 | 0 | 0 |
| - | Indole (DPG 10%) | 5 | 0 | 0 | 0 | 0 |
| A | Musk ketone | 3 | 0 | 3 | 3 | 3 |
| A | "GALAXOLIDE" | 185 | 0 | 185 | 185 | 185 |
| B-i | "FRUITATE" | 0 | 0 | 0 | 5 | 5 |
| B-i | Tricyclodecenyl propionate | 0 | 0 | 0 | 20 | 20 |
| B-i | Methyl salicylate (DPG 10%) | 0 | 0 | 0 | 5 | 5 |
| B-i | Cis-3-hexenyl salicylate | 0 | 0 | 0 | 15 | 15 |
| B-i | Ethyl 2-methylbutyrate (DPG 10%) | 0 | 0 | 0 | 5 | 5 |
| B-ii | o-t-Butyl cyclohexylacetate | 0 | 0 | 0 | 15 | 15 |
| B-iii | G-Decanolactone | 0 | 20 | 0 | 20 | 20 |
| B-iv | "DAMASCENONE" | 0.5 | 0 | 0 | 0.5 | 0.5 |
| B-iv | "d-DAMASCONE" | 1.5 | 0 | 0 | 1.5 | 1.5 |
| B-iv | β-Ionon | 20 | 0 | 0 | 20 | 20 |
| B-iv | Methylionon-G | 25 | 0 | 0 | 25 | 25 |
| B-v | α-Methyl-1,3-benzodioxol-5-propanal | 0 | 0 | 10 | 10 | 10 |
| B-v | Decanal (DPG 10%) | 0 | 0 | 2 | 2 | 2 |
| B-v | Dodecanal (DPG 10%) | 0 | 0 | 1 | 1 | 1 |
| B-v | Hexylcinnamic aldehyde | 0 | 0 | 90 | 90 | 90 |
| B-v | α-Methyl-4-(1-methylethyl)-benzenepropanal | 0 | 0 | 15 | 15 | 15 |
| B-v | p-t-Butyl-α-methylhydrocinnamic aldehyde | 0 | 0 | 140 | 140 | 140 |
| C | Limonene | 25 | 0 | 0 | 25 | 25 |
| D | p-Menthane-8-thiol-3-one (1% DPG) | 0 | 0 | 0 | 0 | 1 |
| Ether | Dodecahydro-3a,6,6,9a-tetramethyl-naphtho[2.1-b]furan | 0 | 0 | 0 | 0 | 0.3 |
| | FORMULA*^{note 2} | 340 | 340 | 340 | 340 | 340 |
| Solvent | Dipropylene glycol | 388 | 640 | 402 | 57 | 55.7 |
| | Total (parts by weight) | 1000 | 1000 | 1000 | 1000 | 1000 |

| | | | | | | |
|---|---|---|---|---|---|---|
| FORMULA*^{note 2} Fragrance formula formed primarily of alcohols and containing solvents such as dipropylene glycol, etc. | | | | | | |

The results are shown in Table 4.

**Table 4**

| Results of Ranking in Stability and Masking Effect | | | | | | | | | | | | | |
|---|---|---|---|---|---|---|---|---|---|---|---|---|---|
| | | Comp. Exs. | | Examples | | | | | Comp. Exs. | | Examples | | |
| | | 1 | 2 | 1* | 2* | 3* | 4 | 5 | 3 | 4 | 6* | 7 | 8 |
| Composition of Table 1 | | 99.50% | 99.50% | 99.50% | 99.50% | 99.50% | 99.50% | 99.50% | 99.50% | 99.50% | 99.50% | 99.50% | 99.50% |
| Fragrance composition 1 | | 0.50% | | | | | | | | | | | |
| Fragrance composition 2 | | | 0.50% | | | | | | | | | | |
| fragrance composition 3 | | | | 0.50% | | | | | | | | | |
| Fragrance composition 4 | | | | | 0.50% | | | | | | | | |
| Fragrance composition 5 | | | | | | 0.50% | | | | | | | |
| fragrance composition 6 | | | | | | | 0.50% | | | | | | |
| Fragrance composition 7 | | | | | | | | 0.50% | | | | | |
| Fragrance composition 8 | | | | | | | | | 0.50% | | | | |
| Fragrance composition 9 | | | | | | | | | | 0.50% | | | |
| Fragrance composition 10 | | | | | | | | | | | 0.50% | | |
| fragrance composition 11 | | | | | | | | | | | | 0.50% | |
| Fragrance composition 12 | | | | | | | | | | | | | 0.50% |
| | | | | | | | | | | | | | |
| changes with time | Room temp. 1day | 1 | 3 | 3.5 | 4 | 3.5 | 4 | 5 | 4 | 2 | 4 | 5 | 5 |
| | 50°C 1 month later | <1 | 2 | 3 | 4 | 3.5 | 3.5 | 4.5 | 1 | 2 | 3.5 | 4.5 | 4.5< |
| Performance | | 1 | 3 | 3.5 | 4 | 3.5 | 4 | 5 | 4 | 2 | 4 | 5 | 5 |
| (masking performance) | | | | | | | | | | | | | |

| | | | | | | | | | | | | | |
|---|---|---|---|---|---|---|---|---|---|---|---|---|---|
| * Reference Examples | | | | | | | | | | | | | |

As shown in FIG. 4, the conditioning shampoos formulated by fragrancing the aliquots of the unfragranced conditioning shampoo of Table 1 with the fragrance compositions of Examples 1, 4, 5, 7 and 8 according to the present invention were acknowledged to have clear advantages in changes with time and performance (masking performance) over the conditioning shampoos formulated by fragrancing the aliquots of the unfragranced conditioning shampoo of Table 1 with the fragrance compositions of Comparative Examples 1 to 4 from a comparison therebetween.

In the case of Example 5, the change with time upon elapsed time of 1 month at 50°C were ranked "4. 5" as compared with "5" one day after the formulation owing to the inclusion of the fragrance composition 7 according to the present invention, thereby also demonstrating excellent stability at high temperatures.

### Example B Clear Shampoo

| | (wt.%) |
|---|---|
| Sodium POE(2) lauryl ether sulfate | 10.0 |
| Myristyl alcohol | 0.5 |
| Cationized hydroxyethylcellulose | 0.2 |
| Laurylamidopropyl betaine | 0.5 |
| Cocoyl monoethanolamide | 0.3 |
| Malic acid | 0.75 |
| Glycerol | 1.0 |
| Deionized water | Balance |

A clear shampoo-formulated by adding the fragrance composition 7 to the above-described composition (pH 3.7) such that its content became 0.5 wt.% was free of any acid smell, and retained the fragrance over a long time.

### Example C Anti-dandruff Shampoo

| | (wt. %) |
|---|---|
| Sodium POE(2) lauryl ether sulfate | 10.0 |
| Sodium lauryl sulfate | 5.5 |
| Myristyl alcohol | 1.0 |
| Cetanol | 0.5 |
| Cationized hydroxyethylcellulose | 0.3 |
| Cationized guar gum . | 0.3 |
| Cocoyl monoethanolamide | 0.5 |
| Dimethicone (polymerization degree:2,000) | 0.5 |
| Dimethicone (polymerization degree:200) | 0.5 |
| Malic acid | 0.7 |
| Benzyloxyethanol | 0.5 |
| Ethylene glycol distearate | 3.0 |
| Cocoyl benzalconium chloride | 0.5 |
| Glycerol | 1.0 |

### Industrial Applicability

The fragrance compositions to be used according to the present invention are excellent inmasking effects for a smellpeculiar to hair cosmetic compositions having a pH of 1 to 5, and are also superb in long-term stability under high-temperature conditions. Acidic hair cosmetic compositions with the fragrance compositions added therein can each retain a good scent over a long period.

A well-balanced addition of the ingredients (E) to (G) to a fragrance composition makes it possible to obtain a fragrance composition for a hair cosmetic composition having a pH of 1 to 5, which can stably retain a good scent in the hair cosmetic composition having a pH of 1 to 5, gives off a good scent upon application of the hair cosmetic composition, is excellent in the persistence of scent after the application, and moreover, gives off a good scent in a bottled state (from a bottle mouth).

## Claims

1. A hair cosmetic composition having a pH of 1 to 5, which comprises the following ingredients (I) to (IV):
(I) from 1 to 50 wt.% of an anionic surfactant;
(II) from 0.05 to 10 wt.% of oil;
(III) from 0.05 to 10 wt.% of an organic acid; and
(IV) a fragrance composition comprising the following ingredient (A), (B) and (C):
(A) from 0.1 to 70 wt.%, with respect to the total weight of the fragrance composition, of a musk;
(B) from 0.001 to 80 wt.%, with respect to the total weight of the fragrance composition, of a compound represented by the following formula (1) :
R¹-COOR² (1)
wherein R¹ represents a linear, branched or cyclic hydrocarbon group having from 2 to 14 carbon atoms, which may contain an oxygen atom or nitrogen atom in at least one carbon-to-carbon bond thereof; and R² represents a linear, branched or cyclic hydrocarbon group having from 1 to 15 carbon atoms, which may contain an oxygen atom or nitrogen atom in at least one carbon-to-carbon bond thereof; and
(C) from 0.01 to 90 wt.%, with respect to the total weight of the fragrance composition, of a hydrocarbon having a total carbon number of from 5 to 15.

2. The hair cosmetic composition according to Claim 1, further comprising from 0.01 to 20 wt.% of an aromatic alcohol.

3. The hair cosmetic composition according to Claim 1 or 2, wherein the organic acid is lactic acid or malic acid.

4. The hair cosmetic composition according to any one of Claims 1-3, wherein the fragrance composition further comprises, as ingredient (D), from 0.00001 to 1 wt.% of a sulfur-containing compound.

## Patentansprüche

1. Haarkosmetikzusammensetzung mit einem pH von 1 bis 5, umfassend die folgenden Bestandteile (I) bis (IV):
(I) von 1 bis 50 Gew.% eines anionischen Tensides;
(II) von 0,05 bis 10 Gew.% eines Öls;
(III)von 0,05 bis 10 Gew.% einer organischen Säure; und
(IV) eine Duftzusammensetzung, umfassend die folgenden Bestandteile (A), (B) und (C):
(A) von 0,1 bis 70 Gew.%, in Bezug auf das Gesamtgewicht der Duftzusammensetzung, eines Moschus;
(B) von 0,001 bis 80 Gew.% in Bezug auf das Gesamtgewicht der Duftzusammensetzung einer Verbindung mir der folgenden Formel (1):
R¹-COOR² (1)
worin R¹ eine lineare, verzweigte oder cyclische Kohlenwasserstoffgruppe mit 2 bis 14 Kohlenstoffatomen ist, die ein Sauerstoff-oder Stickstoffatom in zumindest einer Kohlenstoff-Kohlenstoff-Bindung davon enthalten kann, und R² eine lineare, verzweigte oder cyclische Kohlenwasserstoffgruppe mit 1 bis 15 Kohlenstoffatomen ist, die ein Sauerstoff-oder Stickstoffatom in zumindest einer Kohlenstoff-Kohlenstoff-Bindung davon enthalten kann; und
(C) von 0,01 bis 90 Gew.%, in Bezug auf das Gesamtgewicht der Duftzusammensetzung, eines Kohlenwasserstoffes mit einer gesamten Kohlenstoffzahl von 5 bis 15.

2. Haarkosmetikzusammensetzung nach Anspruch 1, weiterhin umfassend 0,01 bis 20 Gew.% eines aromatischen Alkohols.

3. Haarkosmetikzusammensetzung nach Anspruch 1 oder 2, worin die organische Säure Milchsäure oder Äpfelsäure ist.

4. Haarkosmetikzusammensetzung nach einem der Ansprüche 1 bis 3, worin die Duftzusammensetzung weiterhin als Bestandteil (D) von 0,00001 bis 1 Gew.% einer schwefelhaltigen Verbindung enthält.

## Revendications

1. Composition cosmétique pour les cheveux ayant un pH de 1 à 5, qui comprend les ingrédients (I) à (IV) suivants :
(I) de 1 à 50% en poids d'un tensioactif anionique ;
(II) de 0,05 à 10% en poids d'huile ;
(III) de 0,05 à 10% en poids d'un acide organique ; et
(IV) une composition de fragrance comprenant les ingrédients (A), (B) et (C) suivants :
(A) de 0,1 à 70% en poids, par rapport au poids total de la composition de fragrance, d'un musc ;
(B) de 0,001 à 80% en poids, par rapport au poids total de la composition de fragrance, d'un composé représenté par la formule suivante (1) .
R¹-COOR² (1)
dans laquelle R¹ représente un groupement hydrocarbure à chaîne droite ou ramifiée, ou cyclique ayant de 2 à 14 atomes de carbone, qui peut contenir un atome d'oxygène ou un atome d'azote dans au moins une liaison carbone-carbone de celui-ci, et R² représente un groupement hydrocarbure à chaîne droite ou ramifiée, ou cyclique ayant de 1 à 15 atomes de carbone, qui peut contenir un atome d'oxygène ou un atome d'azote dans au moins une liaison carbone-carbone de celui-ci ; et
(C) de 0,01 à 90% en poids, par rapport au poids total de la composition de fragrance, d'un hydrocarbure ayant un nombre total de carbones de 5 à 15.

2. Composition cosmétique pour les cheveux selon la revendication 1, comprenant en outre de 0,01 à 20% en poids d'un alcool aromatique.

3. Composition cosmétique pour les cheveux selon la revendication 1 ou 2, dans laquelle l'acide organique est l'acide lactique ou l'acide malique.

4. Composition cosmétique pour les cheveux selon l'une quelconque des revendications 1 à 3, dans laquelle la composition de fragrance comprend en outre, comme ingrédient (D), de 0,00001 à 1% en poids d'un composé contenant du soufre.
